# FASCICULE DE BREVET EUROPEEN

(11) **EP 1 607 070 B1**
(45) Date de publication et mention de la délivrance du brevet: **04.05.2011**
(21) Numéro de dépôt: 05356106.4
(22) Date de dépôt: 14.06.2005
(51) Int. Cl.: A61F 2/40

(54) **Composant glénoïdien de prothése d'épaule, jeu d'éléments constitutifs d'un tel composant et prothése totale d'épaule incorporant un tel composant**
Gelenkpfannenteil einer Schulterprothese, Satz von solch einen Teil aufbauenden Elementen und Schultertotalprothese mit einem solchen Teil
Glenoid component of a shoulder prosthesis, set of elements making up such a component and total shoulder prosthesis comprising such a component

(30) Priorité: 15.06.2004 FR 0406473
(43) Date de publication de la demande: 21.12.2005
(73) Titulaire: TORNIER, 38330 Saint-Ismier (FR)
(72) Inventeur: Tornier, Alain, 38330 Saint Ismier (FR); Sirveaux, François, 54600 Villers les Nancy (FR); Walsch, Gilles, 69003 Lyon (FR); Mole, Daniel, 54000 Nancy (FR); Levigne, Christophe, 69300 Caluire (FR); Boileau, Pascal, 06200 Nice (FR); Favard, Luc, 37270 Montlouis (FR)
(74) Mandataire: Myon, Gérard Jean-Pierre

(56) Documents cités:
- EP-A- 0 903 127
- EP-A- 1 064 890
- WO-A-01/47442
- WO-A-93/09733
- WO-A-02/067821
- FR-A- 2 545 352
- FR-A- 2 737 107
- FR-A- 2 835 425
- US-A- 3 978 528
- US-A1- 2004 220 673

## Description

L'invention a trait à un composant glénoïdien de prothèse d'épaule ainsi qu'à un jeu d'éléments constitutifs d'un tel composant et à une prothèse d'épaule incorporant un tel composant.

Dans le domaine des prothèses d'épaule, il est connu par exemple de US-A-3,978,528, de constituer une prothèse dite « inversée » dans laquelle une surface articulaire convexe solidaire de la glène et une surface articulaire concave solidaire de l'humérus coopèrent pour recréer une articulation au niveau de l'épaule. Dans ce genre de prothèse, le composant glénoïdien peut être formé, ainsi qu'il ressort par exemple de FR-A-2 835 425 ou de WO-A-01/47442, d'une embase destinée à être immobilisée sur la glène et d'un élément destiné à être monté sur cette embase et définissant la surface d'articulation convexe.

EP-A-1488764 (voir aussi EP-A-1 598 034), qui appartient à l'état de la technique selon l'article 54(3), divulgue une prothèse inversée pour l'articulation de l'épaule dont le composant glénoïdien est formé d'une embase, d'une tête glénoïdienne et d'un pion intercalé entre cette embase et cette tête.

L'invention propose un nouveau composant glénoïdien de prothèse d'épaule qui incorpore une embase pouvant être à symétrie de révolution, donc particulièrement aisée à installer, mais qui permet également d'adapter la position de la surface articulaire convexe du composant glénoïdien à son environnement, notamment à la position de la surface articulaire humérale concave, tout en facilitant le travail du muscle deltoïde pour limiter, autant que faire se peut, les efforts à développer par un patient pour soulever son bras équipé d'une telle prothèse.

Dans cet esprit, l'invention concerne un composant glénoïdien de prothèse d'épaule selon la revendication 1.

Grâce à l'invention, la position du centre de symétrie de la portion sphérique de la surface articulaire convexe peut ne pas être alignée avec l'axe central de l'embase, ce qui autorise un ajustement satisfaisant de la position de la prothèse par rapport à l'omoplate.

Selon des aspects avantageux mais non obligatoires, un composant glénoïdien peut incorporer une ou plusieurs des caractéristiques suivantes prises dans toute combinaison techniquement admissible :
- L'élément précité définit un logement de déception au moins partiel de la partie axisymétrique de l'embase, ce logement étant globalement centré sur un autre axe qui est décalé par rapport au centre géométrique précité et, en configuration assemblée du composant, sensiblement confondu avec l'axe précité de l'embase.
- La surface périphérique du logement est globalement tronconique et divergente en direction de son embouchure, avec un demi-angle au sommet de valeur sensiblement égale à celle du demi-angle au sommet de la partie précitée de l'embase.
- En configuration implantée du composant sur la glène, le centre géométrique précité est décalé vers le bas du corps du patient par rapport au deuxième axe. Ceci permet notamment d'éviter les interférences entre le composant huméral de la prothèse et le pilier de l'omoplate à la fin du mouvement d'adduction.
- L'élément précité est pourvu d'une face arrière dans laquelle débouche un logement de réception au moins partielle de la partie axisymétrique de l'embase, alors que ce logement est centré sur un axe confondu avec l'axe de la partie symétrique en configuration assemblée du composant.
- L'élément définit une surface de raccordement entre la partie globalement en portion de sphère de la surface articulaire et une portion de la périphérie de cette face arrière.
- La partie en portion de sphère est géométriquement centrée sur un axe parallèle à l'axe central de la partie précitée de l'embase.
- En variante, la partie en portion de sphère est géométriquement centrée sur un axe qui n'est pas perpendiculaire à une face arrière du composant destinée à venir en appui contre la glène du patient. Cet aspect de l'invention permet de contrôler l'orientation du premier axe et de le positionner d'une façon correcte, y compris lorsque la surface fraisée de la glène n'est pas parallèle à un plan vertical contenant la colonne vertébrale du patient debout, ce qui arrive notamment lorsque la partie supérieure de l'omoplate est usée ou détruite.

L'invention concerne également un jeu d'éléments tel que celui mentionné ci-dessus qui sont aptes à être rapportés chacun, pour constituer un élément glénoïdien de prothèse d'épaule, sur une embase elle-même apte à être immobilisée sur la glène d'un patient, chaque élément définissant une surface d'articulation convexe dont une partie au moins est globalement en portion de sphère centrée sur un point géométrique, alors que chaque élément forme un logement globalement axisymétrique de réception au moins partielle d'une partie de l'embase et que le décalage entre le centre géométrique précité et l'axe de symétrie du logement est variable d'un élément à l'autre. Ce jeu d'éléments permet au chirurgien de choisir l'élément dont la géométrie est la plus adaptée une fois que l'embase a été immobilisée sur la glène du patient.

L'invention concerne également une prothèse totale d'épaule qui comprend un composant glénoïdien tel que mentionné ci-dessus ou dont l'élément qui forme la surface d'articulation convexe a été sélectionné dans un jeu d'éléments tel que mentionné ci-dessus.

L'invention concerne enfin une méthode de pose d'un composant glénoïdien de prothèse d'épaule qui peut être mise en oeuvre avec un composant tel que décrit ci-dessus et, plus spécifiquement une méthode comprenant des étapes consistant à :
- immobiliser sur la glène du patient l'embase du composant glénoïdien ;
- sélectionner, parmi plusieurs éléments aptes à être rapportés sur cette embase et dont les centres géométriques des parties de surfaces articulaires en portion de sphère sont décalés différemment par rapport à un axe central d'un logement de réception au moins partielle d'une partie de cette embase, un élément dont le centre géométrique de la partie de surface en portion de sphère sera, une fois monté sur l'embase, dans une position prédéterminée et
- monter l'élément sélectionné sur l'embase.

L'invention sera mieux comprise et d'autres avantages de celle-ci apparaîtront plus clairement à la lumière de la description qui va suivre d'un mode de réalisation d'un composant glénoïdien et d'une prothèse conformes à son principe, donnée uniquement à titre d'exemple et faite en référence aux dessins annexés dans lesquels :
- la figure 1 est une représentation schématique de principe d'une prothèse d'épaule conforme à l'invention implantée sur un patient ;
- la figure 2 est une vue éclatée, partiellement en coupe, d'un composant glénoïdien de la prothèse de la figure 1 ;
- la figure 3 est une vue en perspective d'un élément constitutif du composant glénoïdien de la figure 2 ;
- la figure 4 est une vue analogue à la figure 3 pour un deuxième élément susceptible d'appartenir à un composant glénoïdien tel que représenté à la figure 2 et
- la figure 5 est une vue analogue à la figure 3 pour un troisième élément susceptible d'appartenir à un composant glénoïdien tel que représenté à la figure 2.

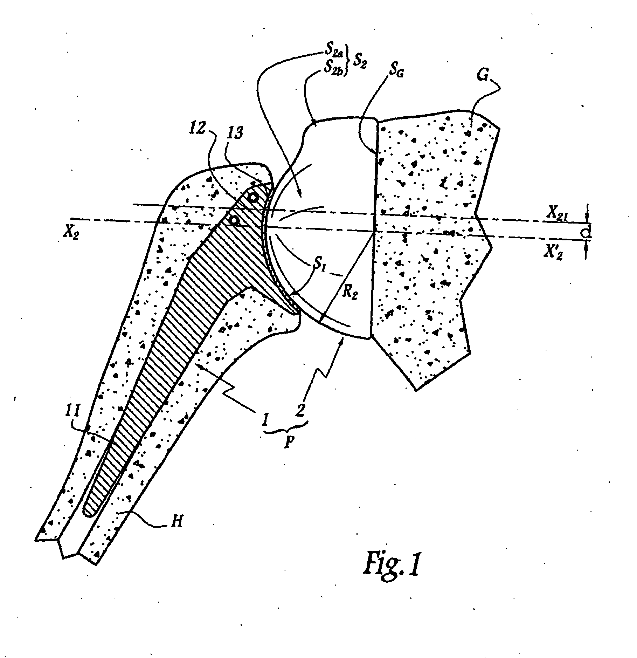

La prothèse P représentée à la figure 1 comprend un composant huméral 1 comportant une tige 11 destinée à être ancrée dans le canal médullaire de l'humérus H, ainsi qu'une partie métaphysaire 12 dans laquelle est immobilisée une coupelle 13 en polyéthylène définissant une surface articulaire concave S₁ en forme de tronçon de sphère.

La prothèse P comprend également un composant glénoïdien 2 qui définit une surface articulaire convexe S₂ et qui est destinée à être implantée sur la glène G de l'épaule après que celle-ci ait été fraisée pour créer une surface S_{G} globalement parallèle à un plan vertical, non représenté, contenant la colonne vertébrale du patient lorsqu'il se tient debout.

Pour la clarté du dessin, le composant 1 est représenté en coupe, alors que le composant 2 est représenté en vue extérieure sur la figure 1.

La surface S₂ comprend une portion S₂ₐ globalement en forme de demi-sphère, centrée sur un point géométrique C₂ et de rayon R₂.

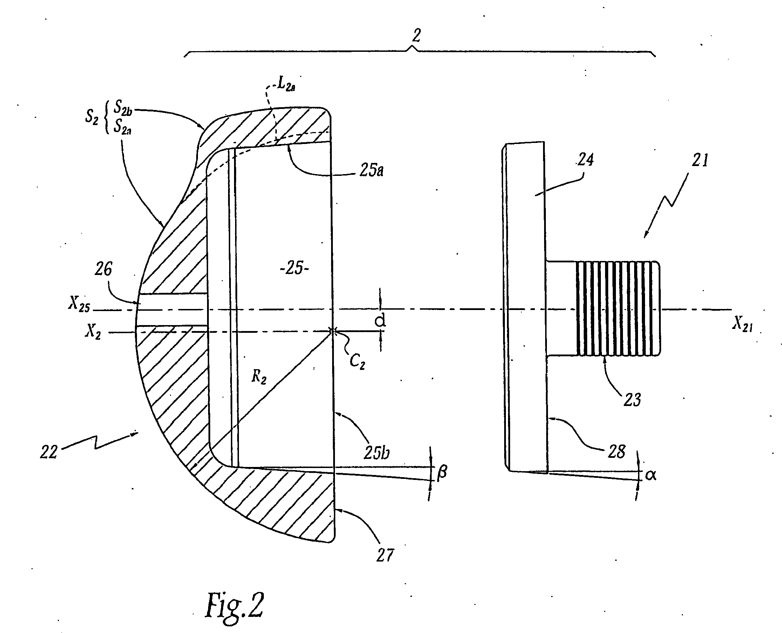

Comme il ressort plus particulièrement de la figure 2, le composant glénoïdien 2 est formé par l'assemblage de deux parties, à savoir une embase 21 et un élément 22, parfois dénommé « tête », qui définit la surface S₂ et qui est destiné à être monté sur l'embase 21 lorsque celle-ci a été ancrée sur la glène G.

L'embase 21 est globalement à symétrie de révolution et on note X₂₁ son axe central. L'embase 21 comprend une queue d'ancrage 23 destinée à être introduite dans un perçage correspondant à réaliser dans la glène G ainsi qu'une partie 24 destinée à faire saillie par rapport à la surface S_{G} lorsque l'embase 21 est implantée. La partie 24 est tronconique et l'on note α son demi-angle au sommet.

A la figure 2, l'embase 21 est représentée en vue extérieure, alors que la tête 22 est représentée en coupe dans un plan de symétrie.

La tête 22 définit un logement 25 de réception de la partie 24 lorsque la tête 22 est montée sur l'embase 21. Ce logement 25 est centré sur un axe de symétrie X₂₅ qui est confondu avec l'axe X₂₁ en configuration assemblée du composant 2.

La surface périphérique 25a du logement 25 est globalement tronconique et divergente en direction de son embouchure 25b, avec un demi-angle au sommet β de valeur sensiblement égale à celle du demi angle α, ce qui permet d'obtenir une immobilisation de la tête glénoïdienne 22 sur l'embase 21 à la façon d'un cône Morse.

Un perçage 26 centré sur l'axe X₂₅ permet d'accéder au logement 25 par l'extérieur, c'est-à-dire à l'opposé de la surface 27 de la tête 22 qui est normalement tournée vers la surface S_{G} fraisée dans la glène. Cet orifice 26 permet au chirurgien de manipuler un moyen de serrage de la tête 22 sur l'embase 21 tel que connu, par exemple, de FR-A-2 835 425.

Le centre C₂ de la surface S₂ n'est pas aligné sur l'axe X₂₅ mais décalé de celui-ci d'une distance d non nulle. On note X₂ un axe perpendiculaire à la surface 27 et passant par le centre C₂. Cet axe est un axe de symétrie de la partie sphérique S₂ₐ de la surface S₂ prolongée par une surface imaginaire dont la trace est représentée par la ligne pointillée L₂ₐ en arc de cercle à la figure 2. On peut en effet considérer une surface hémisphérique imaginaire congruente à la partie S₂ₐ. La trace de cette surface à la figure 2 serait un arc de cercle centré sur le point C₂ et formé par la réunion de l'arc de cercle représentant la partie S₂ₐ et par la ligne L₂ₐ. L'axe X₂ serait alors un axe de symétrie de cette surface.

L'axe X₂ est parallèle à l'axe X₂₅ et, en configuration montée de la tête 22, à l'axe X₂₁.

Le centre C₂ définit la position de la partie S₂ₐ de la surface S₂ qui est celle qui interagit effectivement avec la surface S₁ du composant huméral 1.

Lorsque le composant glénoïdien 2 est assemble, le centre C₂ est décalé par rapport à l'axe X₂₁ de la distance d qui est non nulle, ce qui permet d'abaisser la partie active S₂ₐ de la surface S₂ pour un meilleur positionnement de la prothèse par rapport à l'omoplate du patient.

Comme la portion S₂ₐ de la surface S₂ ne permettrait pas à la tête 22 d'inclure le logement 25 compte tenu du décalage d, une surface de transition gauche S_{2b} prolonge la portion S₂. jusqu'au bord de la surface 27 dans sa partie la plus éloignée du centre C₂. Le fait que la surface S_{2b} n'est pas hémisphérique ne gêne pas le fonctionnement de la prothèse P dans la mesure où cette surface n'interagit normalement pas avec la surface S₁.

A ce sujet, on peut imaginer, dans le cadre de la présente invention, que la tête 22 soit tronquée dans sa partie supérieure représentée à la figure 2, c'est-à-dire que la surface 25a soit interrompue dans sa partie qui dépasse de la ligne L₂ₐ à la figure 2, puisque cette partie n'interagit normalement pas avec la surface S₁.

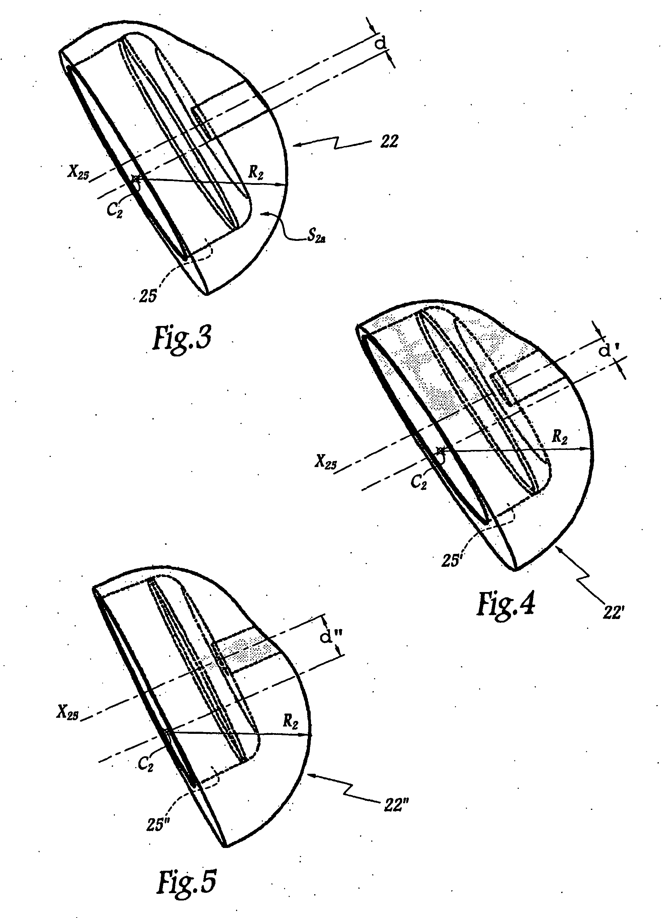

Comme il ressort plus particulièrement de la comparaison des figures 3 à 5, différentes têtes 22, 22' et 22" peuvent être montées sur l'embase 21, ces têtes ayant des décalages d, d' et d" différents entre les centres géométriques C₂ des portions hémisphériques des surfaces S₂ qu'ils définissent et les axes centraux X₂₅ des logements 25, 25' et 25" qu'ils définissent également. Les décalages d, d', d" correspondent, une fois l'une de ces têtes montées sur une embase, aux décalages entre les centres C₂ et l'axe X₂₁.

Ainsi, les trois têtes glénoïdiennes représentées respectivement aux figures 3 à 5 constituent un jeu d'éléments pouvant être sélectivement rapporté sur une embase 21 et dont l'un peut être sélectionné par le chirurgien après que l'embase a été ancrée sur la glène pour que son centre C₂ soit dans une position pré-déterminée qui tient compte de la géométrie de la glène et/ou de la position de la surface articulaire concave S₁ de l'élément huméral.

La pose d'une prothèse conforme à l'invention permet donc au chirurgien, après avoir ancré l'embase 21 du composant glénoïdien, d'ajuster la position de la surface articulaire convexe S₂ par un choix raisonné de la tête glénoïdienne 22, 22' ou 22", ceci afin d'améliorer le confort du patient.

Lorsque ce choix a été effectué, il suffit au chirurgien de monter la tête sélectionnée sur l'embase et de l'immobiliser par tout moyen approprié.

Selon une variante non représentée de l'invention, l'axe X₂ de la partie hémisphérique S₂ₐ de la surface S₂ peut ne pas être perpendiculaire à la face arrière 28 de la partie 24 ou à la surface 27 de la tête 22 qui viennent en appui ou sont parallèles à la surface S_{G}. Ceci permet de corriger un défaut de parallélisme entre la surface S_{G} et le plan précité contenant la colonne vertébrale du patient debout, grâce à un « déversement » de la partie hémisphérique de la surface S₂ par rapport à la surface S_{G}.

## Revendications

1. Composant glénoïdien (2) de prothèse d'épaule comprenant une embase (21), apte à être immobilisée sur la glène (G) d'un patient, et un élément (22) apte à être rapporté sur ladite embase et définissant une surface d'articulation convexe (S₂) dont une partie au moins (S₂ₐ) est globalement en portion de sphère et centrée sur un point géométrique (C₂), ledit élément (22) étant pourvu d'une face arrière (27) dans laquelle débouche un logement (25) de réception au moins partielle d'une partie (24) de l'embase centrée sur un axe (X₂₁) et sur laquelle est rapporté ledit élément, alors que, en configuration assemblée dudit composant (2), ledit centre géométrique (C₂) et ledit axe (X₂₁) sont décalés (d) l'un par rapport à l'autre, et alors que ledit élément (22) définit une surface de raccordement (S_{2b}) prolongeant la partie (S₂ₐ) globalement en portion de sphère de la surface articulaire (S₂) jusqu'au bord (27a) de la face arrière (27) dans sa partie la plus éloignée du point géométrique (C₂) sur lequel est centrée la portion de surface (S₂ₐ) en portion de sphère.

2. Composant selon la revendication 1, **caractérisé en ce que** ledit élément (22) définit un logement (25) de réception au moins partielle de ladite partie (24) de ladite embase (21), ledit logement étant globalement centré sur un axe (X₂₅) qui est décalé (d) par rapport audit centre géométrique (C₂) et, en configuration assemblée dudit composant (2), sensiblement confondu avec ledit axe (X₂₁) de ladite partie (24) de ladite embase (21).

3. Composant selon la revendication 2, **caractérisé en que** la surface périphérique (25a) du logement (25) est globalement tronconique et divergente en direction de son embouchure (25b), avec un demi-angle au sommet (β) de valeur sensiblement égale à celle du demi-angle au sommet (α) de ladite partie (24) de l'embase (21).

4. Composant selon l'une des revendications précédentes, **caractérisé en ce que** le sens de décalage entre ledit centre géométrique (C₂) et ledit axe (X₂₁) est tel que, en configuration implantée dudit composant (2) sur la glène (G) ; ledit centre est décalé vers le bas du corps du patient par rapport audit axe.

5. Composant selon l'une des revendications précédentes, **caractérisé en ce que** ledit logement (25) de réception au moins partielle de ladite partie (24) de ladite embase (21) est centré sur un axe (X₂₅) confondu avec ledit axe (X₂₁) de ladite partie (24) de ladite embase (21) en configuration assemblée dudit composant.

6. Composant selon l'une des revendications précédentes, **caractérisé en ce que** ladite partie (S₂ₐ) en portion de sphère est géométriquement centrée sur un axe (X₂) qui est parallèle à l'axe central (X₂₁) de ladite partie (24) de ladite embase.

7. Composant selon l'une des revendications 1 à 5, **caractérisé en ce que** ladite partie (S₂ₐ) en portion de sphère est géométriquement centrée sur un axe (X₂) non perpendiculaire à une face arrière (27, 28) dudit composant (2) destinée à venir en appui contre la glène (S_{G}) du patient.

8. Jeu d'éléments (22, 22', 22'') aptes à être rapportés chacun, pour constituer un élément glénoïdien (2) de prothèse d'épaule, sur une embase (21) elle-même apte à être immobilisée sur la glène (G) d'un patient, chaque élément définissant une surface d'articulation convexe (S₂) dont une partie au moins (S₂ₐ) est globalement en portion de sphère centrée sur un point géométrique (C₂), alors que chaque élément définit une surface de raccordement (S_{2b}) prolongeant la partie (S₂ₐ) globalement en portion de sphère de la surface articulaire jusqu'au bord (27a) d'une face arrière (27) de cet élément, dans sa partie la plus éloignée du point géométrique (C₂), alors que chaque élément, forme un logement (25) globalement axisymétrique de réception au moins partielle d'une partie (24) de ladite embase et alors que le décalage (d, d', d'') entre ledit centre géométrique (C₂) et l'axe de symétrie (X₂₅) du logement est variable d'un élément à l'autre.

9. Prothèse totale d'épaule, **caractérisée en ce qu'**elle comprend un composant glénoïdien (2) selon l'une des revendications 1 à 7 ou dont l'élément (22) formant la surface d'articulation convexe (S₂) appartient à un jeu d'éléments (22, 22', 22'') selon la revendication 8.

## Claims

1. Glenoidal component (2) of a shoulder prosthesis comprising a base (21) adapted to be immobilized on the glenoid (G) of a patient, and an element (22) adapted to be added on said base and defining a convex surface of articulation (S₂) of which at least a part (S₂ₐ) is globally in the form of a portion of sphere and centred on a geometric point (C₂), said element (22) being provided with a rear face (27) into which opens out a housing (25) for at least partially receiving a part (24) of said base centred on an axis (X₂₁) and on which said element is added, whereas, when said component (2) is in assembled configuration, said geometric centre (C₂) and said axis (X₂₁) are offset (d) with respect to each other, and said element (22) defines a connection surface (S_{2b}) which extends the part (Sₐ) globally in the form of a portion of sphere of the articular surface (S₂) up to the edge (27a) of the rear face (27) in its part most remote from the geometric point (C₂) on which the part (S₂ₐ) in the form of a position of a sphere is centred.

2. The component of claim 1, wherein said element (22) defines a housing (25) for at least partially receiving said part (24) of said base (21), said housing being globally centred on an axis (X₂₅) which is offset (d) with respect to said geometric centre (C₂) and, when the component (2) is in assembled configuration, substantially merges with said axis (X₂₁) of said part (24) of said base (21).

3. The component of claim 2, wherein the peripheral surface (25a) of the housing (25) is globally frustro-conical and divergent in the direction of its mouth (25b), with a semi-vertex angle (β) of a value substantially equal to that of the semi-vertex angle (α) of said part (24) of the base (21).

4. The component of one of previous claims, wherein the direction of offset between said geometric centre (C₂) and said axis (X₂₁) is such that, when said component (2) is in implanted configuration on the glenoid (G), said centre is offset downwardly of the patient's body with respect to said axis..

5. The component of one of the previous claims, wherein said housing (25) for at least partially receiving said part (24) of said base (21) is centred on an axis (X₂₅) which merges with said axis (X₂₁) of said part (24) of said base (21) when said component is in assembled configuration.

6. The component of one of the previous claims, wherein said part (S₂ₐ) in the form of a portion of sphere is geometrically centred on an axis (X₂) which is parallel to the central axis (X₂₁) of said part (24) of said base.

7. The component of one of the claims 1 to 5, wherein said part (S₂ₐ) in the form of a portion of sphere is geometrically centred on an axis (X₂) non perpendicular to a rear face (27, 28) of said component (2) intended to come into abutment against the patient's glenoid (S_{G}).

8. Set of elements (22, 22', 22") each adapted to be added, in order to constitute a glenoidal element (2) of a shoulder prosthesis, on a base (21) itself adapted to be immobilized on the glenoid (G) of a patient, each element defining a convex surface of articulation (S₂) of which at least a part (S₂ₐ) is globally in the form of a portion of sphere centred on a geometric point (C₂), while each element defines a connection surface (S_{2b}) which extends the part (S₂ₐ) globally in the form of a portion of a sphere of the articular surface up to the edge (27a) of a rear face (27) of this element, in its part most remote from the geometrical point (C₂), while each element forms a globally axisymmetric housing (25) for at least partially receiving a part (24) of said base, and the offset (d, d', d") between said geometric centre (C₂) and the axis of symmetry (X₂₅) of the housing is variable from one element to the other.

9. Total shoulder prosthesis, wherein it comprises a glenoidal component (2) of one of the claims 1 to 7 or whose element (22) forming the convexe articulation surface (S₂) belongs to a set of element (22, 22', 22") according to claim 8.

## Patentansprüche

1. Glenoidkomponente (2) einer Schulterprothese, die eine Grundplatte (21), die auf der Gelenkpfanne (G) eines Patienten fixierbar ist, und ein Element (22) aufweist, das auf die Grundplatte aufsetzbar ist und eine konvexe Gelenkfläche (S₂) definiert, von der mindestens ein Bereich (S₂ₐ) im Wesentlichen die Form eines Kugelabschnitts hat und auf einen geometrischen Punkt (C₂) zentriert ist, wobei das Element (22) mit einer Rückseite (27) versehen ist, in die ein Sitz (25) zur zumindest teilweisen Aufnahme eines Bereichs (24) der Grundplatte mündet, der auf eine Achse (X₂₁) zentriert ist und auf den das Element aufgesetzt wird, während in der zusammengesetzten Konfiguration der Komponente (2) die geometrische Mitte (C₂) und die Achse (X₂₁) zueinander versetzt sind (d), und während das Element (22) eine Anschlussfläche (S_{2b}) definiert, die den im Wesentlichen kugelabschnittförmigen Bereich (S₂ₐ) der Gelenkfläche (S₂) bis zum Rand (27a) der Rückseite (27) in ihrem am weitesten vom geometrischen Punkt (C₂) entfernten Bereich verlängert, auf den der kugelabschnittförmige Flächenbereich (S₂ₐ) zentriert ist.

2. Komponente nach Anspruch 1, **dadurch gekennzeichnet, dass** das Element (22) einen Sitz (25) zur zumindest teilweisen Aufnahme des Bereichs (24) der Grundplatte (21) definiert, wobei der Sitz im Wesentlichen auf eine Achse (X₂₅) zentriert ist, die bezüglich der geometrischen Mitte (C₂) versetzt ist (d) und in der zusammengesetzten Konfiguration der Komponente (2) im Wesentlichen mit der Achse (X₂₁) des Bereichs (24) der Grundplatte (21) zusammenfällt.

3. Komponente nach Anspruch 2, **dadurch gekennzeichnet, dass** die Umfangsfläche (25a) des Sitzes (25) im Wesentlichen kegelstumpfförmig ist und in Richtung ihrer Mündung (25b) divergiert, mit einem halben Scheitelwinkel (β) eines Werts der im Wesentlichen gleich demjenigen des halben Scheitelwinkels (α) des Bereichs (24) der Grundplatte (21) ist.

4. Komponente nach einem der vorhergehenden Ansprüche, **dadurch gekennzeichnet, dass** die Versatzrichtung zwischen der geometrischen Mitte (C₂) und der Achse (X₂₁) derart ist, dass in der auf die Gelenkpfanne (G) implantierten Konfiguration der Komponente (2) die Mitte bezüglich der Achse auf dem Körper des Patienten nach unten versetzt ist.

5. Komponente nach einem der vorhergehenden Ansprüche, **dadurch gekennzeichnet, dass** der Sitz (25) zur zumindest teilweisen Aufnahme des Bereichs (24) der Grundplatte (21) auf eine Achse (X₂₅) zentriert ist, die in der zusammengesetzten Konfiguration der Komponente mit der Achse (X₂₁) des Bereichs (24) der Grundplatte (21) zusammenfällt.

6. Komponente nach einem der vorhergehenden Ansprüche, **dadurch gekennzeichnet, dass** der kugelabschnittförmige Bereich (S₂ₐ) geometrisch auf eine Achse (X₂) zentriert ist, die parallel zur Mittelachse (X₂₁) des Bereichs (24) der Grundplatte liegt.

7. Komponente nach einem der Ansprüche 1 bis 5, **dadurch gekennzeichnet, dass** der kugelabschnittförmige Bereich (S₂ₐ) geometrisch auf eine Achse (X₂) zentriert ist, die nicht lotrecht zu einer Rückseite (27, 28) der Komponente (2) ist, die dazu bestimmt ist, gegen die Gelenkpfanne (S_{G}) des Patienten in Auflage zu kommen.

8. Satz von Elementen (22, 22', 22"), die jeweils zur Bildung eines Glenoidelements (2) einer Schulterprothese auf eine Grundplatte (21) aufsetzbar sind, die selbst auf der Gelenkpfanne (G) eines Patienten fixiert werden kann, wobei jedes Element eine konvexe Gelenkfläche (S₂) definiert, von der mindestens ein Bereich (S₂ₐ) im Wesentlichen die Form eines auf einen geometrischen Punkt (C₂) zentrierten Kugelabschnitts hat, während jedes Element eine Anschlussfläche (S_{2b}) definiert, die den im Wesentlichen kugelabschnittförmigen Bereich (S₂ₐ) der Gelenkfläche bis zum Rand (27a) einer Rückseite (27) dieses Elements in ihrem am weitesten vom geometrischen Punkt (C₂) entfernten Bereich verlängert, während jedes Element einen im Wesentlichen achssymmetrischen Sitz (25) zur zumindest teilweisen Aufnahme eines Bereichs (24) der Grundplatte bildet, und während der Versatz (d, d', d") zwischen der geometrischen Mitte (C₂) und der Symmetrieachse (X₂₅) des Sitzes von einem Element zum anderen variabel ist.

9. Schultertotalprothese, **dadurch gekennzeichnet, dass** sie eine Glenoidkomponente (2) nach einem der Ansprüche 1 bis 7 enthält oder deren die konvexe Gelenkfläche (S₂) formendes Element (22) zu einem Satz von Elementen (22, 22', 22") nach Anspruch 8 gehört.
